# EUROPÄISCHE PATENTSCHRIFT

(11) **EP 1 697 293 B1**
(45) Veröffentlichungstag und Bekanntmachung des Hinweises auf die Patenterteilung: **13.08.2008**
(21) Anmeldenummer: 04804574.4
(22) Anmeldetag: 25.11.2004
(51) Int. Cl.: C07C 49/587, C07C 45/66, C07C 45/64, C07C 49/487, A61K 8/18, C11B 9/00

(54) **(Z)-7-CYCLOHEXADECEN-1-ON ALS RIECHSTOFF**
USE OF (Z)-7-CYCLOHEXADECENE-1-ONE AS A PERFUME
(Z)-7-CYCLOHEXADECENE-1-ONE COMME PARFUM

(30) Priorität: 23.12.2003 DE 10361524
(43) Veröffentlichungstag der Anmeldung: 06.09.2006
(73) Patentinhaber: Symrise GmbH & Co. KG, 37603 Holzminden (DE)
(72) Erfinder: RECKZIEGEL, Aurelia, 41540 Dormagen (DE); WÖHRLE, Ingo, 37603 Holzminden (DE); SONNENBERG, Steffen, 04416 Markkleeberg (DE)
(74) Vertreter: Eisenführ, Speiser & Partner
(86) Internationale Anmeldenummer: PCT/EP2004/053102
(87) Internationale Veröffentlichungsnummer: WO 2005/063670

(56) Entgegenhaltungen:
- US-A- 2 790 005
- MATHUR, H. AND BHATTACHARYYA, S.: "Macrocyclic musk compounds-IX New syntheses of cyclohexadecenone and cyclohexadecanone from aleuritic acid" TETRAHEDRON, Bd. 21, 1965, Seiten 1537-1540, XP002230767

## Beschreibung

Die vorliegende Erfindung betrifft primär die neue Verbindung (Z)-7-Cyclohexadecen-1-on, Riechstoff- und Aromastoffmischungen umfassend (E,Z)-7-Cyclohexadecen-1-on, deren jeweilige Verwendung als Riech- oder Aromastoff(mischung), entsprechende parfümierte Produkte sowie Verfahren zur Herstellung von 7-Cyclohexadecen-1-on.

In der Parfümindustrie besteht generell ein Bedarf an Moschusriechstoffen, da den Konsumenten laufend neue und moderne Düfte mit Moschusduft zur Verfügung gestellt werden sollen. Riechstoffe mit Moschusgeruch werden in großer Menge und ungezählten Variationen in Parfüms, Riechstoffmischungen (Parfümkompositionen) und Parfümierungen für die verschiedensten Anwendungsgebiete eingesetzt. Wegen der steigenden Nachfrage der Verbraucher nach neuen modernen Duftnoten, besteht in der Parfümindustrie ein ständiger Bedarf an Duftstoffen, mit denen sich in Parfüms neuartige Effekte erzielen und auf diese Art neue Modetrends kreieren lassen. Verbindungen mit Moschusgeruch sind seit jeher wichtige und begehrte Komponenten in der Duftstoffindustrie. Somit kommen heutzutage Moschusriechstoffe in vielen Parfümkompositionen zum Einsatz.

Typische makrocyclische Moschusriechstoffe zeichnen sich durch einen Ring mit 13 bis 17 C-Atomen aus, welcher als funktionelle Gruppe ein Keton oder einen Ester trägt. Klassische Moschusriechstoffe sind z.B. Zibeton, Muscon, Cyclopentadecanolid, Ethylenbrassilat und Cylopentadecanon. Parfümeure sprechen allgemein bei diesen Moschuskörpern von einem "Makromoschusgeruch", wobei die einzelnen Verbindungen sich in einzelnen Noten und Aspekten zum Teil sehr deutlich voneinander unterscheiden.

Für die Kreation neuartiger moderner Kompositionen besteht ständiger Bedarf an Moschusriechstoffen mit besonderen geruchlichen Eigenschaften, die geeignet sind, als Grundlage für die Komposition von neuartigen modernen Parfüms mit komplexem Moschuscharakter zu dienen. Die gesuchten Moschusriechstoffe sollen neben dem typischen Moschusgeruch weitere Noten und Aspekte aufweisen, die ihnen geruchlichen Charakter und Komplexität verleihen.

Die Suche nach geeigneten Moschusriechstoffen, die zur vorliegenden Erfindung führte, wurde durch folgende Sachverhalte erschwert:
- Die Mechanismen der Geruchswahmehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Moschusriechstoffen hängt deshalb stark von der Intuition des Suchenden ab.

Es war daher die Aufgabe der vorliegende Erfindung, makrocyclische Moschusverbindungen mit neuen Geruchseigenschaften zu finden, mit welchen Riechstoffkompositionen besondere geruchliche Noten und Aspekte verliehen werden können.

Es wurde nun überraschenderweise gefunden, dass (Z)-7-Cyclohexadecen-1-on geeignet ist, die gestellte Aufgabe zu lösen.

Im J. Am. Chem. Soc., 1955, 77, 5423 bzw. US 2,790,005 wurde aus 1,9-Cyclohexadecandion durch partielle Reduktion und anschließende Dehydratisierung eine Mischung aus (E,Z)-8-Cyclohexadecen-1-on hergestellt, die einen "intensiven Moschusgeruch" aufwies.

J. Chem. Soc. 1965, 6679 beschreibt die Synthese von reinem (Z)-8-Cyclohexadecen-1-on durch Hydrierung von 8-Cyclohexadecin-1-on. (Z)-8-Cyclohexadecen-1-on wird als "eher schwerer Moschus" beschrieben.

In DE 2 111 753 und J. Org. Chem., 1972, 37, 3846 werden makrocyclische Verbindungen und Verfahren zu ihrer Herstellung beschrieben. Es wird pauschal ausgeführt, dass diese makrocyclischen Verbindungen einen "Moschusduft" besitzen, welcher üblicherweise als die animalische Note in Parfüms betrachtet wird. Ein Verfahren zur Herstellung (E,Z)-8-Cyclohexadecen-1-on ausgehend von 1,9-Cyclohexadecadien wird beschrieben durch Monoepoxidierung, Reduktion und Oxidation. Eine detailliertere Geruchsbeschreibung findet sich hier jedoch nicht.

Die Synthese eines Gemisches von (E)-7-Cyclohexadecen-1-on und (E)-8-Cyclohexadecen-1-on ist in Tetrahedron, 1965, 21, 1537 beschrieben. Aleuritinsäure (9,10,16-Trihydroxypalmitinsäure) wird als Isopropylidenderivat geschützt, zur Dicarbonsäure oxidiert, hydrobromiert und verestert. Aus dem resultierenden Diester erhält man nach Brom-Eliminierung über weitere Stufen ein Gemisch von (E)-7-Cyclohexadecen-1-on und (E)-8-Cyclohexadecen-1-on. Da die Autoren die pauschale Auffassung äußern, dass der Moschusgeruch der Cyclohexadecenone nicht von der Position der Doppelbindung beeinflusst wird, war ihnen an einer Isolierung und Charakterisierung der beiden einzelnen Isomeren nicht gelegen.

Ein Gemisch bestehend aus (E,Z)-8-Cyclohexadecen-1-on war früher einen begrenzten Zeitraum unter den Namen Animusk^{®} auf dem Markt (Fragrance Resources).

Es hat sich nun überraschenderweise gezeigt, dass sich (Z)-7-Cyclohexadecen-1-on der Formel (1) geruchlich deutlich von (E)-7-Cyclohexadecen-1-on sowie von den Isomeren des 8-Cyclohexadecen-1-on unterscheidet.

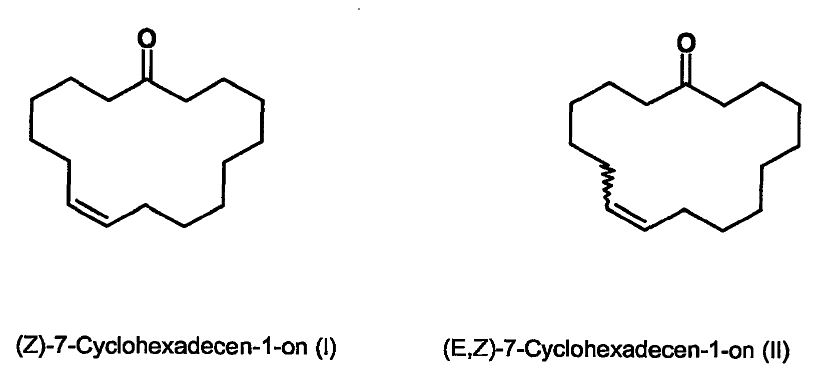

Die Geruchsbeschreibung einzelner Isomere und der Mischungen von (E,Z)-7-und/oder (E,Z)-8-Cyclohexadecen-1-on sind in Tabelle 1 dargestellt.

**Tabelle 1**

| Verbindung(en) | Geruchsbeschreibung |
|---|---|
| (E)-7-Cyclohexadecen-1-on | Angeruch: kräftiger Moschusgeruch, leichte Nitromoschusnote Nachgeruch: insgesamt eher schwach, Musk, etwas erogen, leichte Nitromoschusnote |
| (Z)-7-Cyclohexadecen-1-on Formel (1) | Angeruch: von allen hier untersuchten Isomeren und Mischungen der sauberste, reintönigste und stärkste Moschusgeruch; kristallin und erogen, leicht animalisch (Zibeton-artig) Nachgeruch: von allen Isomeren der intensivste und typischste Moschusgeruch; warm und erogen, mit kristallinen und milden animalischen Tönen |
| (E,Z)-7-Cyclohexadecen-1-on | Angeruch: starke, saubere Moschusnote, kristalline Nitromoschusnote, leicht holziger Aspekt Nachgeruch: warm, erogen, animalisch, kristalline, leicht Nuancen |
| (E)-8-Cyclohexadecen-1-on | Angeruch: starker Moschusgeruch, Nitromoschusnote Nachgeruch: eleganter, klassischer Makromoschuskörper, Nitromoschus, sauber, eher linear |
| (Z)-8-Cyclohexadecen-1-on | Angeruch und Nachgeruch: ähnlich (E)-8-Cyclohexadecen-1-on, insgesamt etwas schwächer, dafür animalische (Muscon-hafte) Nuancen |
| (E,Z)-8-Cyclohexadecen-1-on | Angeruch: eleganter, starker Moschusgeruch mit kräftiger Nitromoschusnote Nachgeruch: eleganter, klassischer Makromoschuskörper, Nitromoschus, balsamisch, sauber, eher linear |
| Mischung aus (E,Z)-7-und (E,Z)-8-Cyclohexadecen-1-on | Angeruch: kräftiger, sauberer und komplexer Moschusgeruch, ausgeprägte Nitromoschusnote, fein blumiger und süßer sowie pudriger Aspekt, schwach holziger Akzent Nachgeruch: sauberer, komplexer, starker und exaltierender Makromoschusgeruch, warm, leicht kristallin, erogen, animalisch, sehr naturhaft und abgerundet, schwach holzige Nuance |

Die geruchlich bewerteten Mischungen waren wie folgt zusammengesetzt (Werte gemäß GC-Analytik):
(E,Z)-7-Cyclohexadecen-1-on der Formel (II) enthielt 77,7% (E)-7-Cyclohexadecen-1-on und 21,8% (Z)-7-Cyclohexadecen-1-on der Formel (I)
(E,Z)-8-Cyclohexadecen-1-on enthielt 71,5% (E)-8-Cyclohexadecen-1-on und 28% (Z)-8-Cyclohexadecen-1-on.

Die Mischung aus (E,Z)-7- und (E,Z)-8-Cyclohexadecen-1-on enthielt gemäß GC-Analytik 19,8% (E)-7-Cyclohexadecen-1-on, 8,4% (Z)-7-Cyclohexadecen-1-on der Formel (I), 40,6% (E)-8-Cyclohexadecen-1-on und 30,5% (Z)-8-Cyclohexadecen-1-on (vgl. Beispiel 1.2 unten).

Die reinen Isomere (E)-8-Cyclohexadecen-1-on und (Z)-8-Cyclohexadecen-1-on waren mittels Spaltrohrdestillation aus (E,Z)-8-Cyclohexadecen-1-on erhalten worden.

Die hier vorgestellten eingehenderen sensorischen Untersuchungen haben gezeigt, dass die in der Literatur gegebenen eher pauschalen Hinweise zur sensorischen Bewertung der bisher bekannten 7/8-Cyclohexadecenone nicht exakt genug bzw. nicht korrekt sind.

Überraschenderweise unterscheidet sich das bisher unbekannte (Z)-7-Cyclohexadecen-1-on geruchlich deutlich von (E)-7-Cyclohexadecen-1-on und von den Isomeren des 8-Cyclohexadecen-1-ons, insbesondere durch die parfümistisch begehrte und wertvolle kristalline Nitromoschusnote sowie durch den sauberen, reintönigen und intensiven Moschusgeruch.

Im vorliegenden Falle wird dabei unter "kristallin" der typische Nitromoschusgeruch von Musk Ambrette (2,6-Dinitro-3-methoxy-1-methyl-4-tert-butylbenzol) verstanden, der geruchlich durch seinen süßen, blumigen und pudrigen Moschusgeruch charakterisiert ist.

(Z)-7-Cyclohexadecen-1-on zeigt von den hier untersuchten Verbindungen und Mischungen den stärksten und saubersten Moschusgeruch, insbesondere einen Nitromoschusgeruch, der Musk Ambrette ähnlich ist.

Die Kombination von (Z)-7-Cyclohexadecen-1-on (I) und (E)-7-Cyclohexadecen-1-on zu (E,Z)-7-Cyclohexadecen-1-on (II) weist bereits einen komplexeren Moschusgeruch auf als die einzelnen Isomere, und zwar den begehrten kristallinen Nitromoschusgeruch, zu dem ein holziger Aspekt hinzukommt. Bevorzugte Gewichtsverhältnisse von (E)-7-Cyclohexadecen-1-on zu (Z)-7-Cyclohexadecen-1-on liegen im Bereich 6 : 1 bis 1 : 2, besonders bevorzugt im Bereich 4 : 1 bis 1 : 1.

Durch die Kombination von (Z)-7-Cyclohexadecen-1-on (I) und (E)-7-Cyclohexadecen-1-on mit (E,Z)-8-Cyclohexadecen-1-on werden schließlich ganz besondere geruchliche Effekte erzielt. Vorteilhafte Gewichtsverhältnisse von (E,Z)-7-Cyclohexadecen-1-on zu (E,Z)-8-Cyclohexadecen-1-on in der Mischung (E,Z)-7,8-Cyclohexadecen1-on liegen dabei im Bereich 10 : 1 bis 1 : 10, bevorzugt im Bereich 5 : 1 bis 1 : 5 besonders bevorzugt im Bereich 2 : 1 bis 1 : 4.

Die Mischung (E,Z)-7,8-Cyclohexadecen-1-on besitzt im Vergleich zu einer Mischung der reinen trans-Isomeren (E)-7,8-Cyclohexadecen-1-on eine wesentlich größere Intensität, Komplexität, Kristallinität und Eleganz und ist daher besonders für die Verwendung in neuen und modernen ParfümKompositionen geeignet.

Die Mischung (E,Z)-7,8-Cyclohexadecen-1-on zeigt die höchste Komplexität der hier untersuchten Mischungen, und zeichnet sich durch Natürlichkeit und Strahlung aus. Der leicht holzige Akzent harmoniert hervorragend mit dem kristallinen, exaltierenden Moschusgeruch. (E,Z)-7,8-Cyclohexadecen-1-on besitzt zudem im direkten Vergleich zu (E,Z)-8-Cyclohexadecen-1-on einen deutlich niedrigeren Geruchsschwellenwert auf.

In Mischungen mit anderen Riechstoffen vermag das erfindungsgemäße (Z)-7-Cyclohexadecen-1-on bereits in geringen Dosierungen die Intensität einer Riechstoffmischung zu verstärken und das Gesamtbild der Riechstoffmischung geruchlich abzurunden sowie der Mischung mehr Ausstrahlung sowie Natürlichkeit zu verleihen. In höheren Dosierungen kommt der saubere, kräftige Moschusgeruch zum Tragen, der von der kristallinen Nitromoschusnote begleitet ist. Es werden mit (E,Z)-7,8-Cyclohexadecenon an Musk Ambrette erinnernde Effekte erzielt und die Komposition weist einen eleganten, exaltierenden und kristallinen Moschusgeruch auf.

Zusammenfassend besitzen daher die folgenden (erfindungsgemäßen) Verbindungen und Mischungen eine überraschende geruchliche Qualität:
- (Z)-7-Cyclohexadecen-1-on;
- Riech- oder Aromastoffmischungen umfassend (Z)-7-Cyclohexadecen-1-on und einen oder mehrere weitere Riech- oder Aromastoffe, z.B:
- Riech- oder Aromastoffmischungen umfassend (Z)-7-Cyclohexadecen-1-on und (E)-7-Cyclohexadecen-1-on, wobei das Gewichtsverhältnis von (E)-7-Cyclohexadecen-1-on zu (Z)-7-Cyclohexadecen-1-on vorzugsweise im Bereich von 6:1 bis 1:2 liegt, bevorzugt im Bereich von 4:1 bis 1:1.
- Riech- oder Aromastoffmischungen umfassend:
   (Z)-7-Cyclohexadecen-1-on,
   (E)-7-Cyclohexadecen-1-on
   (Z)-8-Cyclohexadecen-1-on und
   (E)-8-Cyclohexadecen-1 -on,
wobei das Gewichtsverhältnis von (E,Z)-7-Cyclohexadecen-1-on zu (E,Z)-8-Cyclohexadecen-1-on vorzugsweise im Bereich 10 : 1 bis 1 : 10, bevorzugt im Bereich 5 : 1 bis 1 : 5, besonders bevorzugt im Bereich 2 : 1 bis 1 : 4.

Bevorzugt sind erfindungsgemäße Riech- oder Aromastoffmischungen (wie oben charakterisiert), in denen das Verhältnis von (E)-7-Cyclohexadecen-1-on zu (Z)-7-Cyclohexadecen-1-on höchstens 50 : 1, vorzugsweise höchstens 10 : 1 beträgt, da ansonsten in manchen Fällen der sensorische Charakter der jeweiligen Mischung nicht den Anforderungen genügt.

Ein mit den vorstehend diskutierten eng verwandter Aspekt der Erfindung betrifft die Verwendung von (Z)-7-Cyclohexadecen-1-on oder einer (Z)-7-Cyclohexadecen-1-on umfassenden Riech- oder Aromastoffmischung (wie oben charakterisiert) als Moschusriech- oder -aromastoff bzw. Moschusriech-oder -aromastoffmischung.

In einem entsprechenden erfindungsgemäßen Verfahren zum Vermitteln, Verstärken oder Modifizieren eines Moschusgeruchs wird eine sensorisch aktive Menge (Z)-7-Cyclohexadecen-1-on oder einer (Z)-7-Cyclohexadecen-1-on umfassenden Riech- oder Aromastoffmischung (wie oben charakterisiert) mit einem Erzeugnis in Kontakt gebracht oder gemischt.

Die Herstellung von 7-Cyclohexadecen-1-on kann vorteilhaft aus dem bekannten 1,8-Cyclohexadecandion durch partielle Reduktion und anschließende saure Dehydratisierung erfolgen (es entsteht ein Gemisch von 7-Cyclohexadecen-1-on und 8-Cyclohexadecen-1-on). Entsprechend kann 8-Cyclohexadecen-1-on aus dem bekannten 1,9- Cyclohexadecandion erhalten werden. Es kann auch ein Gemisch aus 1,8-/1,9-Cyclohexadecandionen eingesetzt werden, was insbesondere zur Herstellung der Mischung (E,Z)-7,8-Cyclohexadecen-1-on vorteilhaft ist. 1,8-/1,9-Cyclohexadecandione sind beispielsweise aus J. Org. Chem., 1968, 33, 4541 und US 3,935,270 bekannt.

Als Produkt der partiellen Reduktion von 1,8- und/oder 1,9-Cyclohexadecandion bildet sich ein Gemisch enthaltend unumgesetztes Diketon der Formel (S1a), das gewünschte Hydroxyketon der Formel (S1b) und das Diol der Formel (S1c).

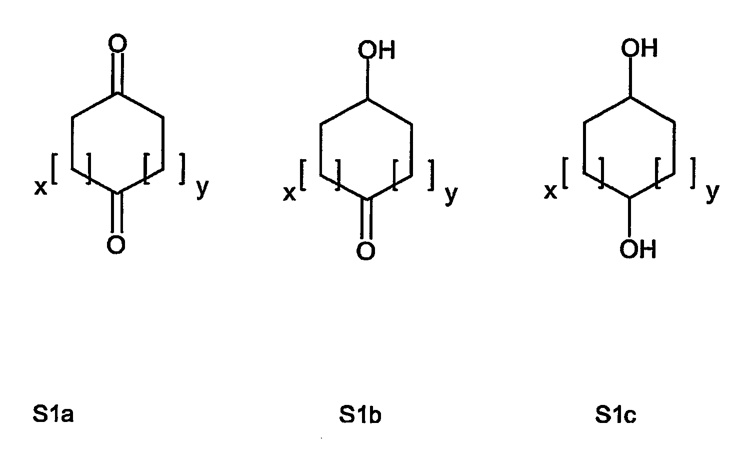

In den Formeln bedeuten, je nach Ausgangsmaterial, (a) x und y = 6 oder (b) x = 5 und y = 7 bedeuten.

Die partielle Reduktion von 1,8- und/oder 1,9-Cyclohexadecandion kann beispielsweise durch Umsetzung mit Metallhydriden oder mittels Hydrierung erfolgen.

Die Umsetzung mit Metallhydriden kann beispielsweise mit Borhydriden, bevorzugt Natriumborhydrid, oder Aluminiumhydriden, dabei vorzugsweise Lithiumaluminiumhydrid oder Diisobutylaluminiumhydrid, erfolgen. Vorteilhafterweise werden 0,30-0,75 molare Äquivalente bezogen auf die Cyclohexadecandion-Menge eingesetzt. Im Falle der Borhydride wird die Reaktion bevorzugt in, gegebenenfalls wässrigen, protischen Lösungsmitteln wie z.B. Ethanol, Methanol oder Isopropanol durchgeführt. Im Falle der Aluminiumhydride wird die Reaktion bevorzugt in aprotischen Lösungsmitteln wie z.B. Diethylether, Tetrahydrofuran oder Toluol durchgeführt.

Bei der Hydrierung ist die katalytische Hydrierung mit Wasserstoff bevorzugt, welche im Regelfall in Gegenwart eines Hydrierkatalysators der achten Nebengruppe des Periodensystems erfolgt, wie z.B. Palladium, Nickel oder Platin. Die erfindungsgemäßen Hydrierkatalys atoren können auf organische oder anorganische Trägermaterialien aufgebracht sein. Die Hydrierkatalysatoren können ein Trägermaterial oder Mischungen von Trägermaterialien enthalten." Als vorteilhafte Trägermaterialen seien Aktivkohle, Kohle, Aluminiumoxide, Metalloxide, Kieselgele, Zeolithe, Tone, Tongranulate oder amorphe Aluminiumsilicate genannt. Bevorzugte Katalysatoren sind Palladium auf Aktivkohle, Raney-Nickel oder Platin-Mohr. Die Hydrierung kann in Lösungsmitteln wie z.B. Ethanol, Isopropanol, Essigester, Hexan, Cyclohexan oder auch lösungsmittelfrei, d.h. in Substanz, erfolgen. Typischerweise erfolgt die Hydrierung bei einen Wasserstoffdruck im Bereich von 1 bis 100 bar, die Temperatur liegt meist im Temperaturbereich von 20 bis 150°C. Das Gewichtsverhältnis des eingesetzten Hydrierkatalysators zu 1,8- und/oder 1,9-Cyclohexadecandion liegt meist im Bereich 0,00001 : 1 bis 0,1 : 1.

Da eine unvollständige Hydrierung zur Bildung der gewünschten Hydroxyketone der Formel (S1b) notwendig ist, ist eine 20-70%ige theoretische Aufnahme an Wasserstoff bezogen auf 1,8- und/oder1,9-Cyclohexadecandion bevorzugt, damit nicht beide Ketogruppen vollständig hydriert werden.

Insbesondere bevorzugt ist die katalytische Hydrierung in Gegenwart von Raney-Nickel als Hydrierkatalysator, gegebenenfalls in Gegenwart katalytischer Mengen (meist 0,05 bis 5 Gew.-% bezogen auf 1,8- und/oder1,9-Cyclohexadecandion) von Basen wie Alkalihydroxiden, z.B. Natriumhydroxid, oder Alkalialkoholaten, z.B. Natriummethanolat. Bevorzugt wird die Hydrierung bei einem Wasserstoffdruck im Bereich von 1 bis 50 bar, bevorzugt 10 bis 20 bar, durchgeführt. Die Temperatur bei der Hydrierung liegt typischerweise im Bereich von 20 bis 100°C, bevorzugt im Bereich von 50 bis 80°C. Bevorzugt ist eine theoretische Wasserstoffaufnahme von 30-60% bezogen auf 1,8-und/oder 1,9-Cyclohexadecandion, bevorzugt eine von 35-55%. Das Gewichtsverhältnis des eingesetzten Hydrierkatalysators zu 1,8- und/oder 1,9-Cyclohexadecandion kann zwischen 0,0001 : 1 bis 0,1 : 1 liegen, bevorzugt ist ein Verhältnis von 0,001 : 1 bis 0,05 : 1. Die Reaktionszeit der Hydrierung beträgt vorteilhafterweise 1 bis 50 Stunden, bevorzugt 6 bis 15 Stunden. Der GC-Gehalt (Gehalt im Gaschromatogramm) der gewünschten Hydroxyketone der Formel (S1b) im Rohprodukt der Hydrierung, liegt meist im Bereich von 30 bis 70 %.

Das Rohprodukt der Hydrierung, das im Wesentlichen aus den Verbindungen (S1a), (S1b) und (S1c) besteht, kann ohne weitere Reinigungsschritte in die Eliminierung (Dehydratisierung) eingesetzt werden. Bei Bedarf kann nach das Rohprodukt nach üblichen Methoden aufgereinigt werden (Destillation, Chromatographie oder auch Kristallisation) und das gereinigte Hydroxyketon der Formel (S1b) in die folgende Reaktionsstufe der Dehydratisierung eingesetzt werden.

In einem zweiten Schritt werden die Hydroxyketone der Formel (S1b) nach dem Fachmann wohlvertrauten Methoden unter sauren Bedingungen dehydratisiert. Geeignete saure Katalyatoren sind beispielsweise konzentrierte Schwefelsäure, p-Toluolsulfonsäure, Benzolsulfonsäure und saure lonentauscher (z.B. aus der Reihe der kommerziell erhältlichen Lewatite^{®} oder Amberlite^{®}).

Nach der Dehydratisierung des Rohproduktes der Hydrierung, von 1,8-und/oder 1,9-Cyclohexadecandion, besteht das resultierende Gemisch im Wesentlichen aus folgenden Produkten: 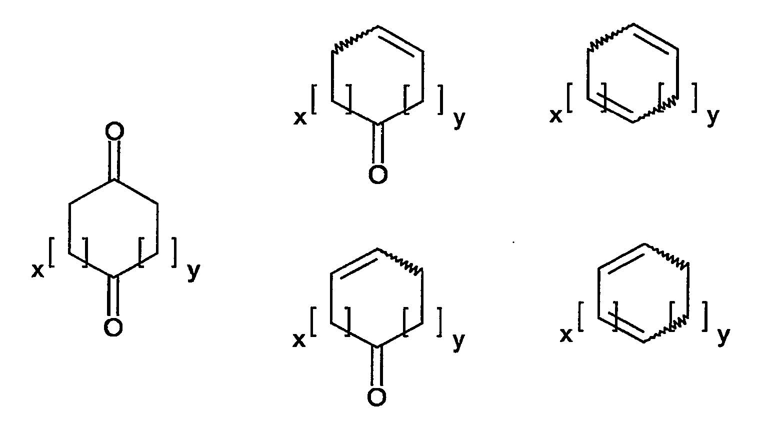 Hierin bedeuten wiederum, je nach Ausgangsmaterial, x und y = 6 oder x = 5 und y = 7.

Ausgehend von reinem 1,9-Cyclohexadecandion erhält man nach partieller Reduktion und Dehydratisierung 1,9-Cyclohexadecandion, (E,Z)-8-Cyclohexadecenon, 1,8-Cyclohexadecadien und 1,9-Cyclohexadecadien.

Ausgehend von reinem 1,8-Cyclohexadecandion erhält man nach partieller Reduktion und Eliminierung 1,8-Cyclohexadecandion, (E,Z)-7-Cyclohexadecenon, (E,Z)-8-Cyclohexadecenon, 1,7-Cyclohexadecadien, 1,8-Cyclohexadecadien und 1,9-Cyclohexadecadien

So wurde ausgehend von einem Gemisch von 1,8- und 1,9-Cyclohexadecandion (das Anteilsverhältnis lag gemäß GC-Analyse bei etwa 1 : 1) nach partieller Reduktion und anschließender Dehydratisierung ein Produkt erhalten, in welchem (E,Z)-7-Cyclohexadecen-1-on und (E,Z)-8-Cyclohexadecen-1-on in einem Verhältnis von etwa 3: 7 enthalten waren.

Es wurde zudem ein alternatives Verfahren zur Herstellung des erfindungsgemäßen (E,Z)-7-Cyclohexadecenons der Formel (II) mit folgenden Schritten gefunden
- Kondensation von 7-Octencarbonsäure mit N,O-Dimethyl-hydroxylamin zum Methoxymethylamid der Oct-7-ensäure,
- Überführen von 1-Brom-9-decen in die entsprechende Grignard-Verbindung,
- Umsetzen des Methoxymethylamids der Oct-7-ensäure mit der Grignard-Verbindung zum 1,17-Octadecadien-8-on und
- Überführen des 1,17-Octadecadien-8-on im Wege einer ringschließenden Olefinmetathese in (E,Z)-7-Cyclohexadecenon.

In einem ersten Schritt wird dabei durch Kondensation von 7-Octencarbonsäure [J. Org. Chem., 1968, 33, 1550-1556] mit N,O-Dimethylhydroxylamin das Methoxymethylamid der Oct-7-ensäure (Weinreb-Amid) [Synthesis, 2000, 1852-1862] dargestellt.

Anschließend wird in die entsprechende Grignard-Verbindung überführtes 1-Brom-9-decen mit obigem Weinreb-Amid zu 1,17-Octadecadien-8-on umgesetzt.

Die Herstellung des erfindungsgemäßen (E,Z)-7-Cyclohexadecenons (II) erfolgt schließlich ausgehend von dem α,ω-ungesättigten Dien 1,17-Octadecadien-8-on in einer ringschließenden Olefinmetathese. Hierzu wird 1,17-Octadecadien-8-on beispielsweise als 0,01 bis 0,001 molare Lösung in Gegenwart von 0,5 bis 10 mol% eines Metathesekatalysators auf Basis von Mo, W oder Ru umgesetzt. Vorteilhaft ist die Umsetzung in Gegenwart von Benzyliden-bis-(tricyclohexylphosphin)-dichlororuthenium (Grubbs-Katalysator) [Synthesis, 1997, 792; Synlett, 1997, 1010].

Das erfindungsgemäße Verfahren kann durch das folgende Formelschema erläutert werden: 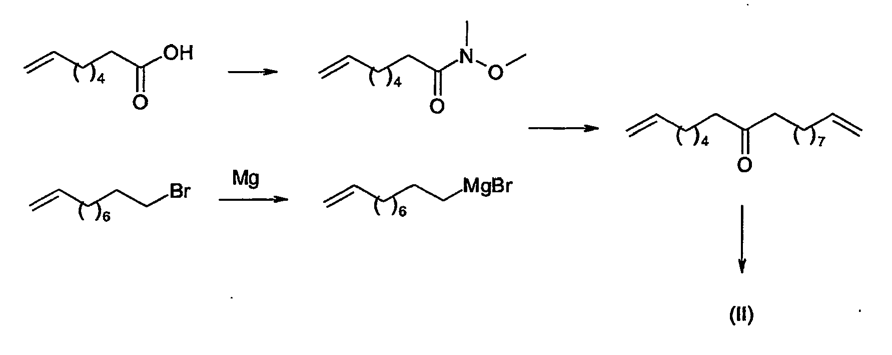 Die Erfindung betrifft auch parfümierte Produkte, umfassend (Z)-7-Cyclohexadecen-1-on oder eine (Z)-7-Cyclohexadecen-1-on umfassende Riech- oder Aromastoffmischung (wie oben charakterisiert).

Übliche sonstige Parfümbestandteile, mit denen (Z)-7-Cyclohexadecen-1-on, (E,Z)-7-Cyclohexadecen-1-on oder (E,Z)-7,8-Cyclohexadecen-1-on vorteilhaft kombiniert werden können , finden sich z.B. in Steffen Arctander, Perfume and Flavor Chemicals, Eigenverlag, Montclair, N.J., 1969; K. Bauer, D. Garbe, H. Surburg, Common Fragrance and Flavor Materials, 4th Edition, Wiley-VCH, Weinheim 2001.

Im Einzelnen seien genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressen-öl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchel-öl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; a Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4.-Decenal; 2-Dodecenal; 2,6,10-Trimethyl-5,9-undecadienal; Heptanaldiethylacetal; 1,1-Dimethoxy-2,2,5-trimethyl-4.-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbon säuren und deren Ester wie z.B. (E)- und (Z)-3-Hexenylformiat; Ethylacetoacetat; Isoamylacetat; Hexylacetat; 3,5,5.Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, lsovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; lsopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; betalsomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-lsocamphylcyclohexanol; 2,6,9-Trimethyl-Z2,Z5,E9-cyclododecatrien-1-ol; 2-Isobutyl-4-methyltetrahydro-2H-pyran-4.-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentyl-cyclohexanon; 5-Cyclohexadecen-1-on; 6,7-Dihydro-1,1,2,3,3-pentamethyl-4(5H)-indanon; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydro-indeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexen-carbaldehyd; 6-lsopropylchinolin; 6-Isobutylchinolin; 6-sec.-Butylchinolin; Indol; Skatol; 2-Methoxy-3-isopropylpyrazin; 2-lsobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die die erfindungsgemäßen (E,Z)-7,8-Cyclohexadecenon, (E,Z)-7-Cylohexadecenon oder (Z)-7-Cyclohexadecenon enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Für manche Anwendungen ist es vorteilhaft, (E,Z)-7,8-Cyclohexadecenon, (E,Z)-7-Cylohexadecenon oder (Z)-7-Cyclohexadecenon enthaltende Parfümöle an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer; Cellulose-basierende Stoffe, Zucker oder Kunststoffe wie PVC, Polyvinylacetate oder Polyurethane sein.

Für andere Anwendungen ist es vorteilhaft, (E,Z)-7,8-Cyclohexadecenon, (E,Z)-7-Cylohexadecenon oder (Z)-7-Cyclohexadecenon enthaltende Parfümöle mikroverkapselt, sprühgetrocknet, als Einschluß-Komplex oder als Extrusions-Produkt einzusetzen und in dieser Form dem zu parfümierenden (Vor-)Produkt hinzufügen.

Die Eigenschaften derart modifizierter Parfümöle werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Die die erfindungsgemäßen Stoffe (E,Z)-7,8-Cyclohexadecenon, (E,Z)-7-Cylohexadecenon oder (Z)-7-Cyclohexadecenon enthaltenden Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver-und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

In Parfümöl-Kompositionen (= Riechstoffmischungen) liegt die eingesetzte Menge von (Z)-7-Cyclohexadecenon üblicherweise im Bereich von 0,001 bis 70 Gew.%, vorzugsweise 0,05 bis 50 Gew.% und besonders bevorzugt 0,5 bis 25 Gew.-%, bezogen auf die gesamte Parfümöl-Komposition.

In Parfümöl-Kompositionen liegt die eingesetzte Menge von (E,Z)-7-Cylohexadecenon oder (E,Z)-7,8-Cyclohexadecenon üblicherweise im Bereich von 0,01 bis 90 Gew.-%, vorzugsweise 0,1 bis 70 Gew.% und besonders bevorzugt 1 bis 40 Gew.%, bezogen auf die gesamte Parfümöl-Komposition.

Zutaten, mit denen die erfindungsgemäßen Stoffe kombiniert werden können, können sind beispielsweise:

Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Mit anderen Moschus-Riechstoffen lassen sich mit (E,Z)-7,8-Cyclohexadecenon, (E,Z)-7-Cylohexadecenon oder (Z)-7-Cyclohexadecenon weitere geruchlich interessante Kombinationen und Effekte erzielen, insbesondere in Kombination mit makrocyclischen Ketonen und insbesondere Lactonen mit Moschusgeruch lassen sich noch facettenreichere Moschusnoten kreieren. Bei den Lactonen sind 1,15-Cyclopentadecanolid, 11-Pentadecen-15-olid, 12-Pentadecen-15-olid, 1,16-Hexadecanolid und Ethylenbrassylat oder deren Gemische bevorzugt zu nennen. Bei den Ketonen sind Muscon, Zibeton, Muscenon, Cyclopentadecanon und Cyclohexadecanon vorteilhaft.

Die erfindungsgemäßen Verbindungen bzw. Mischungen (E,Z)-7,8-Cyclohexadecenon, (E,Z)-7-Cylohexadecenon und (Z)-7-Cyclohexadecenon bzw. die die erfindungsgemäßen Verbindungen bzw. Mischungen (E,Z)-7,8-Cyclohexadecenon, (E,Z)-7-Cylohexadecenon und (Z)-7-Cyclohexadecenon enthaltenden Riechstoff- oder Aromastoffmischungen (wie oben charakterisiert) zeichnen sich durch ein hohes Aufziehvermögen (Eigenhaftung auf einem Substrat) und eine hohe Substantivität (Fähigkeit, aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern).

Besonders bevorzugte erfindungsgemäße parfümierte Produkte sind daher Waschmittel, Hygiene- oder Pflegeprodukte, insbesondere im Bereich der Körperpflege, der Kosmetik und des Haushalts.

Von großem Interesse für die parfümistische Komposition sind daneben Riechstoffe, die die Haftfestigkeit der Komposition verbessern (also als Fixateure wirken) oder die Stärke der geruchlichen Wahrnehmung erhöhen (also als Booster fungieren).

Neben einem hohen Aufziehvermögen zeichnen sich (Z)-7-Cyclohexadecenon und die erfindungsgemäßen Mischungen durch ihren fixierenden Eigenschaften aus. Ein solcher Fixateur erhöht die Haftfestigkeit von anderen Riechstoffen, sei es durch deren Dampfdruckerniedrigung oder geruchlicher Verstärkung (z.B. Absenkung des Schwellenwertes). Die Erfindung betrifft daher auch die Verwendung von (Z)-7-Cyclohexadecen-1-on oder einer (Z)-7-Cyclohexadecen-1-on umfassenden Riechstoff- oder -aromastoffmischung (wie oben charakterisiert) als Fixateur

Des Weiteren wirken (Z)-7-Cyclohexadecenon und die erfindungsgemäßen Mischungen nicht nur als Fixateure, sondern auch als sogenannte Booster oder Enhancer, d.h. sie bewirken eine Verstärkung des Geruchs bzw. der geruchlichen Wahrnehmung von Riechstoffen, Riechstoffmischungen und Parfümkompositionen. Die Erfindung betrifft daher auch die Verwendung von (Z)-7-Cyclohexadecen-1-on oder einer (Z)-7-Cyclohexadecen-1-on umfassenden Riechstöffmischung (wie oben charakterisiert) als Mittel zur Erhöhung der geruchlichen Wahrnehmung von Riechstoffen oder Riechstoffkompositionen.

Die beschriebenen Einflüsse von (Z)-7-Cyclohexadecenon und erfindungsgemäßen Mischungen auf Riechstoffkompositionen zeigen sich besonders bei Vergleich der zeitlichen geruchlichen Veränderung in der Anwendung.

Die folgenden Beispiele erläutern die Erfindung; sofern nicht anders angegeben beziehen sich Anteile und Prozente auf das Gewicht.

### Beispiele:

### Beispiel 1: Herstellung von (E,Z)-7,8-Cyclohexadecen-1-on

### Beispiel 1.1.

3 mol (758 g) einer Mischung von 1,8- und 1,9-Cyclohexadecandion (Isomerenverhältnis 1: 1) wird in 800 ml Isopropanol in Gegenwart von 3 Gew.-% Raney Nickel bei einer Temperatur von 80°C und einem Wasserstoffdruck von 10 bis 20 bar hydriert. Nach Aufnahme von 40% der theoretisch berechneten Wasserstoffmenge wird die Hydrierung abgebrochen, vom Katalysator abfiltriert und der Ansatz vom Lösungsmittel befreit. Man erhält 758 g Rohprodukt mit einer Zusammensetzung (GC-Gehalt): 23% an 8-/9-Dihydroxycylohexadecan, 53% an 8-/9-Hydroxycyclohexadecanon und 23% an 1,8-/1,9-Cyclohexadecandion. Dieses Rohprodukt wird ohne weitere Reinigung in die folgende Dehydratisierung eingesetzt.

### Beispiel 1.2.

In einem 2L-Dreihalskolben, versehen mit Rührer, Innenthermometer, Wasserabscheider und Heizpilz werden das Gemisch aus Beispiel 1.1. in 600 g Toluol vorgelegt, und 20g konz. Schwefelsäure hinzugefügt. Es wird unter ständigem Rühren solange am Wasserabscheider zum Sieden erhitzt, bis kein weiteres Reaktionswasser mehr gebildet wird (abgeschiedenes Wasservolumen: ca. 62 ml). Nach Abkühlung auf Raumtemperatur abgekühlt werden 250 g Wasser, 5 g AcOH und 15 g NaC1 zugesetzt, gerührt, und anschließend die Phasen getrennt. Die organische Phase wird durch vorsichtige Zugabe von 250 ml gesättigter NaHCO₃-Lsg. neutralisiert, das resultierende Gemisch zur besseren Phasentrennung auf 70°C erwärmt, und anschließend weitere 600 g Toluol zugegeben und die Phasen erneut getrennt. Nach Entfernen des Toluols erhält man 574 g Rohprodukt mit einem GC-Gehalt an: Cylohexadecadien von 22%, (E,Z)-7,8-Cyclohexadecen-1-on von 53% und 1,8/1,9-Cyclohexadecandion von 22%. Nach Destillation an einer 7-bödigen Siegwartkolonne erhält man 252 g (E,Z)-7,8-Cyclohexadecen-1-on (Sdp.: 117-120°C, 0,4 mbar), welche folgende Zusammensetzung aufwiesen (GC): 19,8% (E)-7-Cyclohexadecen-1-on, 8,4% (Z)-7-Cyclohexadecen-1-on, 40,6% (E)-8-Cyclohexadecen-1-on und 30,5% (Z)-8-Cyclohexadecen-1-on.

¹H-NMR (400 MHz, CDCl₃): δ (ppm) = 1.30-1.401 (m, 14H), 1.56-1.70 (m, 4H), 2.00-2.08 (m, 4H), 2.32-2.46 (m, 4H),5.24-5.38 (m, 2H).

### Beispiel 2: (E,Z)-7-Cyclohexadecen-1-on

### 1-Brom-9-decen (10-Brom-dec-1-en):

Eine Lösung von 20 g (0,14 mol) Triphenylphosphin in 250 ml Dichlormethan in einer Stickstoffatmosphäre wird auf -10°C gekühlt. Anschließend werden vorsichtig 22,53 g (0,14 mol) Brom zugetropft, bis das Reaktionsgemisch gerade eben eine Gelbfärbung annimmt. Nun werden unter Rühren einige Triphenylphosphin-Kristalle zugesetzt, bis die Gelbfärbung gerade eben verschwindet. Zu der farblosen Suspension werden 20 g (0,12 mol) 10-Decen-1-ol (Quelle: SIGMA-ALDRICH) zugegeben, das Kältebad entfernt und für zwei Stunden bei 20°C gerührt. Anschließend wird vorsichtig mit 50 ml gesättigter NaHCO₃-Lsg. versetzt und mit 100 ml Cyclohexan verdünnt. Die Phasen werden getrennt und die wäßrige Phase dreimal mit je 50 ml Cyclohexan extrahiert. Die vereinigten organischen Phasen werden über Natriumsulfat getrocknet und eingeengt. Der Rückstand wird in Cyclohexan aufgenommen und auf 4°C gekühlt. Es wird von ausgefallenem Triphenylphosphinoxid abfiltriert, der Filterrrückstand mit wenig Cyclohexan gewaschen und das Filtrat erneut eingeengt. Säulenchromatographische Fraktionierung des erhaltenen Rückstands ergibt 24 g (0,11 mol) 1-Brom-9-decen als hellgelbes Öl.

¹H-NMR (400 MHz, CDCl₃): δ (ppm) = 1.30-1.46 (m, 10H), 1.82-1.89 (m, 2H), 2.00-2.08 (m, 2H), 3,405 (t, J = 6,87 Hz, 2H), 4.93 (ddt, J = 10.1, 4.9, 2.23 Hz, 1H), 4.99 (ddt, 17.1, 4.9, 2.18 Hz, 1H), 5.8 (ddt, 17.1, 10.1, 6.56 Hz, 2H).

¹³C-NMR (101 MHz, CDCl₃): δ (ppm) = 28.16, 28.72, 28.87, 29.00, 29.27, 32.83, 33.77, 34.00, 114.19, 139.13.

### Methoxymethylamid der 7-Octensäure:

1,10 g (7,73 mmol) 7-Octensäure [J. Org. Chem., 1968, 33, 1550-1556] werden in 35 ml Dichlormethan gelöst und auf 0°C gekühlt. Anschließend werden 1,75 ml (10,05 mmol) Ethyldiisopropylamin, 935 mg (9,6 mmol) N,O-Dimethylhydroxylamin-Hydrochlorid und 1,63 g (8,50 mmol) N,N-Dicyclohexylcarbodiimid zugegeben. Daraufhin wird der Ansatz auf 0°C abgekühlt und eine katalytische Menge N,N-Dimethylaminopyridin dazugegeben. Das Reaktionsgemisch wird über Nacht bei Raumtemperatur gerührt. Das ausgefallene Harnstoffderivat wird abfiltriert und das Filtrat eingeengt. Der Rückstand wird in Pentan aufgenommen und eventuell ausgefallenes Harnstoffderivat erneut abfiltriert. Die organische Phase wird zweimal mit 0,5M Salzsäure und einmal mit ges. NaHCO₃-Lsg. gewaschen, getrocknet und eingeengt. Säulenchromatographische Fraktionierung ergibt 1 g (5,41 mmol) Methoxymethylamid der Oct-7-ensäure.

¹H-NMR (400 MHz, CDCl₃): δ (ppm) = 1.31-1.47 (m, 4H), 1.64 (q, J = 7.46 Hz, 2H), 2.05 (dt, J = 6.81 , 1.38 Hz, 2H), 2.41 (t, 7.58 Hz, 2H), 3.17 (s, 3H), 3.68 (s, 3H), 4.93 (ddt, J = 10.16, 2.1, 1.23 Hz, 1H), 4.99 (ddt, J = 17.1, 2.1, 1.5 Hz, 1H), 5.80 (ddt, J =17.01, 10.1, 6.7 Hz, 1H).

¹³C-NMR (101 MHz, CDCl₃): δ (ppm) = 24.48, 28.70, 28.92, 31.86, 32.21, 33.63, 61.20, 114.32, 138.97, 174.69.

### 1,17-Octadecadien-8-on :

Aus 2,15 g (8,53 mmol) 10-Brom-dec-1-en und 249 mg (10,22 mmol) Magnesiumpulver in 20 mL Diethylether unter Stickstoff wird eine Grignardlösung bereitet. Die frische Grignard-Lösung wird langsam unter N₂-Atmosphäre bei 0°C in eine Lösung aus 790 mg (4,26 mmol) des Methoxymethylamids der Oct-7-ensäure und 10 mL Diethylether getropft. Danach wird eine Stunde bei 20°C nachgerührt. Der Ansatz wird vorsichtig mit NH₄Cl-Lsg. gequencht und anschließend zweimal mit Diethylether extrahiert. Die organische Phase wird noch einmal mit Wasser gewaschen, getrocknet und eingeengt. Säulenchromatographische Fraktionierung ergibt 820 mg (3,11 mmol) Octadeca-1,17-dien-8-on.

¹H-NMR (400 MHz, CDCl₃): δ (ppm) = 1.25-1.44 (m, 14H), 1.53-1.61 (m, 4H), 2.0-2.08 (m, 4H), 2.37(t, J = 7,4 Hz, 2H), 2.38 (t, J = 7.4 Hz, 2H), 4.92 (ddt, J = 10.2, 2.8, 1.2 Hz, 1 H), 4.93 (ddt, J = 10.2, 2.8, 1.2 Hz, 1 H), 4.98 (ddt, J = 17.1, 2.2, 1.6 Hz, 1 H), 4.99 (ddt, J = 17.1, 2.2, 1.6 Hz, 1 H), 5.79 (ddt, J = 17.1, 10.3, 6.7 Hz, 1 H), 5.80 (ddt, J = 17.1, 10.3, 6.7 Hz, 1 H).

¹³C-NMR (101 MHz, CDCl₃): δ (ppm) = 23.71, 23.88, 28.69, 28.73, 28.92, 29.08, 29.26, 29.32, 29.37, 33.59, 33.80, 42.73, 42.83, 114.15, 114.38, 138.86, 139.17, 211.51.

### (E,Z)-7-Cyclohexadecenon/Isomerentrennung:

Zu einer Lösung aus 820 mg (3,11 mmol) Octadeca-1,17-dien-8-on in 560 ml CH₂Cl₂ werden 200 mg (0,243 mmol) Benzyliden-bis-(tricyclohexylphosphin)-dichlororuthenium (Grubbs-Katalysator), gelöst in 20 ml CH₂Cl₂, zugegeben und 6-8 h unter Rückfluß erhitzt. Nach dem Abkühlen auf Raumtemperatur wird die Reaktionslösung zweimal mit je 100 ml 1N Salzsäure gewaschen, über Na₂SO₄ getroc knet und über eine kurze Kieselgel-Säule filtriert. Nach Entfernung des Lösungsmittels erhält man 670 mg rohes (E,Z)-7-Cyclohexadecen-1-on (Isomerenverhältnis (E) : (Z) etwa 3:1).

300 mg (GC-Reinheit etwa 95%) des (E,Z)-7-Cyclohexadecenons wurden präparativ mittels HPLC getrennt. Man erhält 170 mg (E)-7-Cyclohexadecenon mit einer GC-Reinheit von größer 97% und 55 mg (Z)-Cyclohexadecenon mit einer GC-Reinheit größer 97% sowie 50 mg einer Mischfraktion (E,Z)-7-Cyclohexadecenon.

HPLC-Bedingungen: Säule Saphir 110 Si, 5µm, 125x20mm, Eluent: Heptan/t-Butylmethylether (v/v) 99:01, Fluss: 25 ml/min, Druck: 43 bar, Temperatur: 20°C, Detektion: IR; zusätzliche Detektion: UV bei 285 nm.

(Z)-7-Cyclohexadecenon:

¹H-NMR (400 MHz, CDCl₃): δ (ppm) = 1.24-1.40 (m, 14H), 1.54-1.70 (m, 4H), 1.99-2.07 (m, 4H), 2.39 (t, J = 6,3 Hz, 2H), 2.41 (t, J = 6,3 Hz, 2H), 5.26-5.38 (m, 2H).

¹³C-NMR (101 MHz, CDCl₃): δ (ppm) = 23.44, 24.43, 26.85, 27.00, 27.01, 27.68, 27.93, 28.06, 28.48, 28.68, 29.25, 41.78, 42.00, 130.07, 130.15, 212.36.

IR (cm-¹): 2997, 2925, 2853, 1707, 1459, 1439, 1404, 1369, 1207, 844, 718.

(E)-7-Cyclohexadeoenon:

¹H-NMR (400 MHz, CDCl₃): δ (ppm) = 1.16-1.40 (m, 14H), 1.60-1.65 (m, 4H), 1.98-2.06 (m, 4H), 2.36-2.44 (m, 4H), 5.22-5.31 (m, 2H).

¹³C-NMR (101 MHz, CDCl₃): δ (ppm) = 23.71, 24.21, 26.53, 27.23, 27.77, 28.12, 28.29, 28.31, 29.10, 31.96, 32.48, 42.13, 42.59, 130.92, 131.08, 212.10.

IR (cm⁻¹): 2981, 2925, 2851, 1709, 1457, 1433, 1363, 1262, 1210, 1164, 1132, 1103, 1052, 964.

### Beispiel 3:

Das nachfolgende Parfümöl dient in der Praxis zur Parfümierung vielerlei kosmetischer Produkte.

### Zusammensetzung:

| Ingredienzien | Gewichtsteile |
|---|---|
| 1. Citrophoral Base (Symrise) | 5,0 |
| 2. Aldehyd C10 10 % in BA | 5,0 |
| 3. Aldehyd C11 MOA 10 % in BA | 3,0 |
| 4. Farenal (Symrise) | 3,0 |
| 5. Aldehyd C11 10 % in IPM | 5,0 |
| 6. Citroxal 50 % in DEP | 2,0 |
| 7. trans Hex-2-enol 10 % in BA | 2,0 |
| 8. Vertocitral (Symrise) | 1,0 |
| 9. Linalylacetat | 45,0 |
| 10. Citrylal (Symrise) | 5,0 |
| 11. Mandarinal (Firmenich) | 4,0 |
| 12. Lilial (Givaudan Roure) | 75,0 |
| 13. Lyral (IFF) | 75,0 |
| 14. Profarnesol (Symrise) | 5,0 |
| 15. Nerolidol | 5,0 |
| 16. Linalool | 45,0 |
| 17. Geraniumöl afrikanisch | 5,0 |
| 18. Phenylethylalkohol | 75,0 |
| 19. Geraniol | 15,0 |
| 20. Nerol | 10,0 |
| 21. Hexylzimtaldehyd alpha | 50,0 |
| 22. Methyldihydrojasmonat | 15,0 |
| 23. Benzylsalicylat | 100,0 |
| 24. trans,cis-2-Nonadienol 0,1 % in IPM | 5,0 |
| 25. Allylionon (Givaudan Roure) | 3,0 |
| 26. lsomethylionon gamma | 75,0 |
| 27. Eugenol | 7,0 |
| 28. Cedrylacetat | 40,0 |
| 29. Sandolen (Symrise) | 5,0 |
| 30. Citral | 5,0 |

| | |
|---|---|
| BA = Benzylalkohol; IPM = Isopropylmyristat; DEP = Diethylphtalat | |

Der Zusatz von
a) 55 Gewichtsteilen (E,Z)-7,8-Cyclohexadecenon (Zusammensetzung gemäß Beispiel 1.2; Summe Parfümöl: 700 Gewichtsteile) verleiht der Komposition eine leicht erogene, kristalline Nitromoschusnote, die mit existierenden makrocyclischen Moschusriechstoffen nicht erreicht wird. Weiterhin gewinnt die gesamte Komposition an Natürlichkeit und Strahlung, sie erscheint abgerundeter und eleganter.
b) 355 Gewichtsteilen (E,Z)-7,8-Cyclohexadecenon (Zusammensetzung gemäß Beispiel 1.2; Summe eingesetztes Parfümöl: 1000 Gewichtsteile) führt zu einer deutlich wahrnehmbaren Harmonisierung der Komposition. Darüber hinaus werden mit (E,Z)-7,8-Cyclohexadecenon an Musk Ambrette erinnernde Effekte erzielt und die Komposition weist einen eleganten, exaltierenden und kristallinen Moschusgeruch auf. Hierbei setzt sich besonders der wertvolle Charakter (E,Z)-7,8-Cyclohexadecenon im Vergleich zu Kompositionen mit konventionellen makrocyclischen Moschusriechstoffen durch. (E,Z)-7,8-Cyclohexadecenon verleiht der vorliegenden Komposition eine hervorragende Strahlung und gesteigerte Haftung.

### Beispiele 4 bis 6: Anwendungsbeispiele

Bei den im Folgenden gezeigten Vergleichsexperimenten wurde erfindungsgemäßes (E,Z)-7,8-Cyclohexadecenon (Zusammensetzung gemäß Beispiel 1.2) im Vergleich zu nicht erfindungsgemäßen (E,Z)-8-Cyclohexadecenon (Zusammensetzung: (E)-8-: (Z)-8- Cyclohexadecenon = 2 : 1) in verschiedenen Hygiene- und Waschprodukten sowohl durch mehrere Experten als auch durch eine Gruppe von 30 Laienprüfern (Konsumententest) geprüft und hierbei wesentliche geruchliche Unterschiede festgestellt.

### Beispiel 4: Waschpulver (Powder detergent)

Jeweils 80 g Waschpulver (Zusammensetzung: 9% C₁₂-C₁₃ lineare Na-Alkylbenzolsulfonate, 1.6% C₁₄-C₁₅ Na-Alkylethoxysulfat (EO=0.6), 5.7% C₁₂-C₁₈ Alkylsulfate, 3.3% Polyacrylat (MW = 4.500, 27% Aluminosilicat, 0.6% Natriumsilicat, 28% Natriumcarbonat, 9% Natriumsulfat, 0.2% optischer Aufheller, 1.8% Polyethylenglykol (MW = 4.000), 1% Perborat, 1.1% Enzyme (Lipase, Protease, Cellulase), Wasser q.s.) wurden mit 0,2 g einer 50%igen Lösung in Isopropylmyristat von (E,Z)-7,8-Cyclohexadecenon bzw. (E,Z)-8-Cyclohexadecenon vermischt und die Waschpulver 24 Stunden bei Raumtemperatur gelagert. Anschließend wurden jeweils zwei Handtücher aus Baumwolle und zwei Handtücher aus Mischgewebe separat bei 40°C in Waschmaschinen (Hersteller: Miele) mit den jeweils 80 g Waschpulver gewaschen.

Die so erhaltene feuchte Wäsche wurde geruchlich untersucht und gefunden, dass die erfindungsgemäße Mischung (E,Z)-7,8-Cyclohexadecenon eine deutlich höhere Duftintensität aufwies. Weiterhin konnte die kristalline Nitromoschusnote deutlich ausgeprägter wahrgenommen werden.

Abschließend wurden die einzelnen Handtücher für 24 Stunden auf einer Leine getrocknet. Die parfümistische Bewertung der beiden Handtüchern durch eine Expertengruppe ergab eine deutliche höhere Duftintensität für die erfindungsgemäßen Mischung (E,Z)-7,8-Cyclohexadecenon. Eine Prüfergruppe mit 30 Laienprüfern bevorzugte in einer verdeckten Evaluierung die Handtücher mit der erfindungsgemäßen Mischung (E,Z)-7,8-Cyclohexadecenon eindeutig mit signifikanten Unterschied (p < 0.05).

Bei der Trocknung der feuchten Handtücher in einem handelsüblichen Trockner wurde für die erfindungsgemäßen Moschusriechstoffe die begehrte "warme" Nitromoschusnote gefunden, die auch bei geringer Dosierungen der Mischung (E,Z)-7,8-Cyclohexadecenon eine wertvolle Harmonisierung ergibt.

### Beispiel 5: Weichspüler (Fabric softener)

Jeweils 40 g eines Weichspülers (3-fach Konzentrat, 94% Trinkwasser, 5,5% Quartäre Ethanotaminester (Esterquats; Quarternary - ammonium methosulfate), 0,2% Alkyldimethylbenzylammoniumchlorid (Preventol^{®} R50, Bayer AG) und 0,3% einer blauen Farbstoff-Lösung) wurden mit 0,12 g einer 50%igen Lösung in Isopropylmyristat der Moschusriechstoffmischungen (E,Z)-7,8-Cyclohexadecenon, (E,Z)-8-Cyclohexadecenon und 5-Cyclohexadecenon (Gemisch der (E)- und (Z)-Isomeren) gut vermischt und die Weichspüler 24 Stunden bei Raumtemperatur gelagert. Der pH-Wert des Weichspülerkonzentrates liegt typischerweise im Bereich 2 - 3. Anschließend wurden jeweils drei Handtücher aus Baumwolle und drei Handtücher aus Mischgewebe bei 40°C in Waschmaschinen (Hersteller: Miele) zuerst mit 80 g eines unparfümierten Standardwaschpulvers und nachfolgend separat mit den zu untersuchenden Weichspülern gewaschen.

Die so erhaltene feuchte Wäsche wurde für 24 Stunden auf der Leine getrocknet. Die geruchliche Evaluierung der trockenen Handtücher ergab, dass die erfindungsgemäße Mischung (E,Z)-7,8-Cyclohexadecenon eine deutlich höhere Duftintensität aufwies. Darüberhinaus hielt diese Moschusnote länger und intensiver über mehrere Tage auf den Handtüchern an, worin sich das bessere Aufziehvermögen zeigt.

Die folgende Tabelle zeigt die Ergebnisse für die Intensität und die Präferenz nach der jeweiligen Bewertung der trockenen Handtücher nach der Behandlung mit den Weichspülern, jeweils auf einer Skala von 1 (= sehr schwach bzw. nicht gefällig) bis 9 (= sehr stark bzw. sehr gefällig). In beiden Fällen war die statistische Bewertung signifikant (p < 0.05).

| **Mischung** | **Intensität** | **Präferenz** |
|---|---|---|
| (E,Z)-7,8-Cyclohexadecenon | 4.0 | 5.9 |
| (E,Z)-8-Cyclohexadecenon | 3.1 | 5.4 |
| 5-Cyclohexadecenon | 2.9 | 5.4 |

### Beispiel 6: Shampoo

Jeweils 30 g eines Shampoos (20% Plantacare^{®} PS 10 (Cognis GmbH, Na-Laureth sulfat und Laurylglycosid), 2% Natriumchlorid, 1.3% Citronensäure, 0.5% Dragocid^{®} Liquid (Symrise GmbH & Co. KG, Mischung von Phenoxyethanol, Methyl-, Ethyl- Propyl - und Butylparaben), 76.2% Wasser) wurden mit 0,1 g einer 50%igen Lösung in Isopropylmyristat der Moschusriechstoffe (E,Z)-7,8-Cyclohexadecenon beziehungsweise (E,Z)-8-Cyclohexadecenon gut vermischt und die Shampoos 24 Stunden bei Raumtemperatur gelagert. Der pH-Wert des Shampoos lag bei etwa 6. Anschließend wurde jeweils eine 20 g - Haarsträhne (Echthaar) mit 1 g des jeweiligen Shampoos, welches in 2 g Wasser aufgeschäumt wurde, für 1 Minute separat von Hand gewaschen. Zum Schluss wurden beide Haarsträhnen separat 30 Sekunden unter 30°C warmen Wasser ausgespült.

Die so erhaltenen feuchten Haarsträhnen wurde für 1 Minute mit einem elektrischen Haartrockner auf mittlerer Stufe getrocknet. Die geruchliche Evaluierung der Haarsträhnen ergab, dass die erfindungsgemäße Mischung (E,Z)-7,8-Cyclohexadecenon sowohl auf feuchtem und trockenem Haar intensiver wahrgenommen sowie geruchlich bevorzugt wurde.

Die folgende Tabelle zeigt die Ergebnisse für die Intensität und die Präferenz nach der jeweiligen Bewertung der trockenen bzw. feuchten Haarsträhnen, jeweils auf einer Skala von 1 (= sehr schwach bzw. nicht gefällig) bis 9 (= sehr stark bzw. sehr gefällig). In allen Fällen war die statistische Bewertung hochsignifikant (p < 0.01).

| **Mischung** | **Feuchte Haarsträhne** | | **Trockene Haarsträhne** | |
|---|---|---|---|---|
| | Intensität | Präferenz | Intensität | Präferenz |
| (E,Z)-7,8-Cyclohexadecenon | 3.0 | 5.3 | 2.9 | 5.9 |
| (E,Z)-8-Cyclohexadecenon | 2.1 | 4.3 | 2.2 | 4.8 |

## Patentansprüche

1. (Z)-7-Cyclohexadecen-1-on.

2. Riech- oder Aromastoffmischung umfassend (Z)-7-Cyclohexadecen-1-on und einen oder mehrere weitere Riech- oder Aromastoffe.

3. Riech- oder Aromastoffmischung nach Anspruch 2, umfassend (Z)-7-Cyclohexadecen-1-on und (E)-7-Cyclohexadecen-1-on.

4. Riech- oder Aromastoffmischung nach Anspruch 3, wobei das Gewichtsverhältnis von (E)-7-Cyclohexadecen-1-on zu (Z)-7-Cyclohexadecen-1-on im Bereich von 6:1 bis 1:2 liegt, bevorzugt im Bereich von 4:1 bis 1:1.

5. Riech- oder Aromastoffmischung nach Anspruch 3 oder 4, umfassend:
(Z)-7-Cyclohexadecen-1 -on,
(E)-7-Cyclohexadecen-1-on
(Z)-8-Cyclohexadecen-1-on und
(E)-8-Cyclohexadecen-1-on.

6. Riech- oder Aromastoffmischung nach Anspruch 5, wobei das Gewichtsverhältnis von (E,Z)-7-Cyclohexadecen-1-on zu (E,Z)-8-Cyclohexadecen-1-on im Bereich 10 : 1 bis 1 : 10, bevorzugt im Bereich 5 : 1 bis 1 : 5, besonders bevorzugt im Bereich 2 : 1 bis 1 : 4.

7. Verwendung von (Z)-7-Cyclohexadecen-1-on oder einer (Z)-7-Cyclohexadecen-1-on umfassenden Riech- oder Aromastoffmischung nach einem der Ansprüche 2-6 als Moschusriech- oder -aromastoff bzw. Moschusriech- oder -aromastoffmischung.

8. Verfahren zum Vermitteln, Verstärken oder Modifizieren eines Moschusgeruchs, wobei eine sensorisch aktive Menge (Z)-7-Cyclohexadecen-1-on oder einer (Z)-7-Cyclohexadecen-1-on umfassende Riech- oder Aromastoffmischung nach einem der Ansprüche 2-6 mit einem Erzeugnis in Kontakt gebracht oder gemischt wird.

9. Parfümiertes Produkt, umfassend (Z)-7-Cyclohexadecen-1-on oder eine (Z)-7-Cyclohexadecen-1-on umfassende Riechstoffmischung nach einem der Ansprüche 2-6.

10. Parfümiertes Produkt nach Anspruch 9, wobei das Produkt eine Waschmittel-, Hygiene- oder Pflegeformulierung ist.

11. Verwendung von (Z)-7-Cyclohexadecen-1-on oder einer (Z)-7-Cyclohexadecen-1-on umfassenden Riech- oder Aromastoffmischung nach einem der Ansprüche 2-6 als Fixateur.

12. Verwendung von (Z)-7-Cyclohexadecen-1-on oder einer (Z)-7-Cyclohexadecen-1-on umfassenden Riech- oder Aromastoffmischung nach einem der Ansprüche 2-6 als Mittel zur Erhöhung der geruchlichen Wahrnehmung von Riechstoffen oder Riechstoffkompositionen.

13. Verfahren zur Herstellung von (E,Z)-7-Cyclohexadecen-1-on mit folgenden Schritten:
- partielle Reduktion von 1,8-Cyclohexadecandion zum entsprechenden Hydroxyketon und
- Dehydratisierung des Hydroxyketons zum (E,Z)-7-Cyclohexadecen-1-on.

## Claims

1. (Z)-7-Cyclohexadecen-1-one.

2. A fragrance or flavouring mixture comprising (Z)-7-cyclohexadecen-1-one and one or more other fragrances or flavourings.

3. The fragrance or flavouring mixture according to claim 2, comprising (Z)-7-cyclohexadecen-1-one and (E)-7-cyclohexadecen-1-one.

4. The fragrance or flavouring mixture according to claim 3, wherein the weight ratio of (E)-7-cyclohexadecen-1-one to (Z)-7-cyclohexadecen-1-one is in the range from 6:1 to 1:2, preferably in the range from 4:1 to 1:1.

5. The fragrance or flavouring mixture according to claim 3 or 4, comprising:
(Z)-7-cyclohexadecen-1-one,
(E)-7-cyclohexadecen-1-one,
(Z)-8-cyclohexadecen-1-one and
(E)-8-cyclohexadecen-1-one.

6. The fragrance or flavouring mixture according to claim 5, wherein the weight ratio of (E,Z)-7-cyclo-hexadecen-1-one to (E, Z)-8-cyclohexadecen-1-one is in the range from 10:1 to 1:10, preferably in the range from 5:1 to 1:5, particularly preferably in the range from 2:1 to 1:4.

7. The use of (Z)-7-cyclohexadecen-1-one or of a fragrance or flavouring mixture containing (Z)-7-cyclohexadecen-1-one according to one of claims 2 - 6 as a musk fragrance or flavouring, or as a musk fragrance or flavouring mixture.

8. A method for imparting, intensifying or modifying a musk odour, wherein a sensorially active quantity of (Z)-7-cyclohexadecen-1-one or of a fragrance or flavouring mixture containing (Z)-7-cyclohexadecen-1-one according to one of claims 2 - 6 is brought into contact or mixed with a product.

9. A perfumed product comprising (Z)-7-cyclohexadecen-1-one or a fragrance mixture containing (Z)-7-cyclohexadecen-1-one according to one of claims 2 - 6.

10. The perfumed product according to claim 9, wherein the product is a detergent, hygiene or care formulation.

11. The use of (Z)-7-cyclohexadecen-l-one or of a fragrance or flavouring mixture containing (Z)-7-cyclohexadecen-1-one according to one of claims 2 - 6 as a fixative.

12. The use of (Z)-7-cyclohexadecen-1-one or of a fragrance or flavouring mixture containing (Z)-7-cyclohexadecen-1-one according to one of claims 2 - 6 as a means of increasing the odour perception of fragrances or fragrance compositions.

13. A method for the preparation of (E,Z)-7-cyclohexadecen-1-one having the following steps:
- partially reducing 1,8-cyclohexadecanedione to form the corresponding hydroxyketone and
- dehydrating the hydroxyketone to form (E,Z)-7-cyclohexadecen-1-one.

## Revendications

1. (Z)-7-Cyclohexadécén-1-one.

2. Mélange de substances odorantes ou aromatiques comprenant de la (Z)-7-cyclohexadécén-1-one et une ou plusieurs autres substances odorantes ou aromatiques.

3. Mélange de substances odorantes ou aromatiques selon la revendication 2 comprenant de la (Z)-7-cyclohexadécén-1-one et de la (E)-7-cyclohexadécén-1-one.

4. Mélange de substances odorantes ou aromatiques selon la revendication 3 où le rapport massique de la (E)-7-cyclohexadécén-1-one à la (Z)-7-cyclohexadécén-1-one est situé dans le domaine de 6 : 1 à 1 : 2, de préférence dans le domaine de 4 : 1 à 1 : 1.

5. Mélange de substances odorantes ou aromatiques selon la revendication 3 ou 4 comprenant :
de la (Z)-7-cyclohexadécén-1-one
de la (E)-7-cyclohexadécén-1-one
de la (Z)-8-cyclohexadécén-1-one et
de la (E)-8-cyclohexadécén-1-one.

6. Mélange de substances odorantes ou aromatiques selon la revendication 5 où le rapport massique de la (E,Z)-7-cyclohexadécèn-1-one à la (E,Z)-8-cyclohexadécén-1-one est situé dans le domaine de 10 : 1 à 1 : 10, de préférence dans le domaine de 5 : 1 à 1 : 5, de manière particulièrement préférée dans le domaine de 2 : 1 à 1 : 4.

7. Utilisation de la (Z)-7-cyclohexadécén-1-one ou d'un mélange de substances odorantes ou aromatiques comprenant de la (Z)-7-cyclohexadécén-1-one selon l'une des revendications 2-6 comme substance odorante ou aromatique de type musc ou mélange de substances odorantes ou aromatiques de type musc.

8. Procédé pour conférer, renforcer ou modifier une odeur de musc, où une quantité active du point de vue sensoriel de (Z)-7-cyclohexadécén-1-one ou d'un mélange de substances odorantes ou aromatiques comprenant de la (Z)-7-cyclohexadécén-1-one selon l'une des revendications 2-6 est mise en contact ou mélangée avec un produit.

9. Produit parfumé comprenant de la (Z)-7-cyclohexadécén-1-one ou un mélange de substances odorantes comprenant de la (Z)-7-cyclohexadécén-1-one selon l'une des revendications 2-6.

10. Produit parfumé selon la revendication 9 où le produit est une formulation d'agent de lavage, hygiénique ou de soins.

11. Utilisation de la (Z)-7-cyclohexadécèn-1-one ou d'un mélange de substances odorantes ou aromatiques comprenant de la (Z)-7-cyclohexadécén-1-one selon l'une des revendications 2-6 comme fixateur.

12. Utilisation de la (Z)-7-cyclohexadécén-1-one ou d'un mélange de substances odorantes ou aromatiques comprenant de la (Z)-7-cyclohexadécén-1-one selon l'une des revendications 2-6 comme agent pour augmenter la perception olfactive de substances odorantes ou de compositions de substances odorantes.

13. Procédé pour produire la (E,Z)-7-cyclohexadécén-1-one comportant les étapes suivantes :
- réduction partielle de la 1,8-cyclohexadécanedione en l'hydroxycétone correspondante et
- déshydratation de l'hydroxycétone en la (E,Z)-7-cyclohexadécén-1-one.
